(19) Europäisches Patentamt
European Patent Office
Office européen des brevets

(11) Numéro de publication : **0 063 508**
**B1**

# (12) FASCICULE DE BREVET EUROPÉEN

(45) Date de publication du fascicule du brevet : **13.06.84**

(51) Int. Cl.³ : **C 07 C 131/00**, A 61 K 31/15

(21) Numéro de dépôt : **82400521.9**

(22) Date de dépôt : **23.03.82**

(54) **Dérivés de 4-phénoxy-3-hydroxy-butyramidoxime, leur procédé de préparation et leur utilisation en thérapeutique.**

(30) Priorité : **31.03.81 FR 8106469**

(43) Date de publication de la demande :
**27.10.82 Bulletin 82/43**

(45) Mention de la délivrance du brevet :
**13.06.84 Bulletin 84/24**

(84) Etats contractants désignés :
**AT BE CH DE FR GB IT LI LU NL SE**

(56) Documents cités :
**FR-A- 2 210 388**

(73) Titulaire : **LABORATOIRE L. LAFON Société anonyme dite:**
**1 rue Georges Médéric**
**F-94701 Maisons-Alfort (FR)**

(72) Inventeur : **Lafon, Louis**
**5 rue de l'Alboni**
**F-75016 Paris (FR)**

(74) Mandataire : **Combe, André et al**
**CABINET BEAU DE LOMENIE 55 rue d'Amsterdam**
**F-75008 Paris (FR)**

**EP 0 063 508 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés de 4-phénoxy-3-hydroxy-butyramidoxime en tant que produits industriels. Elle concerne également leur procédé de préparation et leur utilisation en thérapeutique notamment en tant que substances agissant sur le système cardiovasculaire.

On sait que des dérivés de 4-phénoxy-3-hydroxy-butyramidoxime ont déjà été décrits. On connaît en particulier du brevet français n° 71-29.685 le chlorhydrate de 4-(3,5-diméthoxyphénoxy)-3-hydroxy-butyramidoxime, et du brevet français n° 72-44.573 le chlorhydrate de 4-(3,5-diméthylphénoxy)-3-hydroxy-butyramidoxime. On connaît par ailleurs du brevet français n° 70-24.016 le chlorhydrate de 4-(α-naphtyloxy)-3-hydroxy-butyramidoxime. Ces produits connus agissent sur le système cardiovasculaire en tant qu'agents antiarythmisants. Parmi ceux-ci le chlorhydrate de 4-(α-naphtyloxy)-3-hydroxy-butyramidoxime (qui a pour N° de code LL 1530) a été commercialisé en tant que médicament antiarythmisant sous le nom de marque de BRADYL (Dénomination Commune Internationale : NADOXOLOL).

On vient de trouver de façon surprenante que de nouveaux dérivés appartenant à la famille des 4-phénoxy-3-hydroxy-butyramidoximes, qui sont structurellement différents des produits connus (du fait de la présence d'un groupe allyle sur le noyau phényle) présentent des propriétés cardiovasculaires intéressantes sur le plan thérapeutique, notamment dans le traitement des troubles du rythme cardiaque.

Un nouveau dérivé selon l'invention appartenant à la famille des 4-phénoxy-3-hydroxy-butyramidoximes est caractérisé en ce qu'il est choisi parmi l'ensemble constitué par

(i) les 4-(allylphénoxy)-3-hydroxy-butyramidoximes de formule générale :

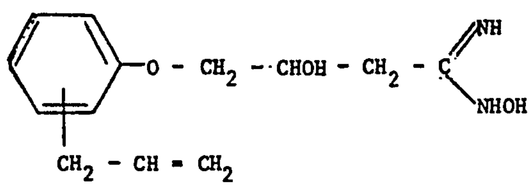

(I)

et,

(ii) leurs sels d'addition.

Par sels d'addition, on entend ici les sels d'addition d'acides (obtenus par réaction d'une base libre de formule I avec un acide minéral ou organique), et les sels d'ammonium. Parmi les acides utilisables pour salifier les bases de formule I, on peut notamment citer les acides chlorhydrique, bromhydrique, nitrique, sulfurique, acétique, propionique, oxalique, fumarique, maléique, succinique, benzoïque, cinnamique, mandélique, citrique, malique, tartrique, p-toluènesulfonique et méthanesulfonique. Parmi les composés permettant d'obtenir des sels d'ammonium, on peut citer notamment $ICH_3$ et $ClCH_3$. Les sels d'addition d'acide sont d'une manière générale préférés aux sels d'ammonium.

L'invention vise donc les 4-(2-allylphénoxy)-, 4-(3-allylphénoxy)- et 4-(4-allylphénoxy)-3-hydroxy-butyramidoximes et leurs sels d'addition. Les produits préférés sur le plan thérapeutique sont le 4-(2-allylphénoxy)-3-hydroxy-butyramidoxime et ses sels d'addition (notamment le chlorhydrate) qui sont particulièrement intéressants en tant qu'agents antiarythmisants.

Les composés de formule I peuvent être préparés selon une méthode connue en soi par application de mécanismes réactionnels classiques. Le procédé préconisé selon l'invention pour préparer un composé de formule I est caractérisé en ce que l'on fait réagir un 4-(allylphénoxy)-3-hydroxy-butyronitrile de formule :

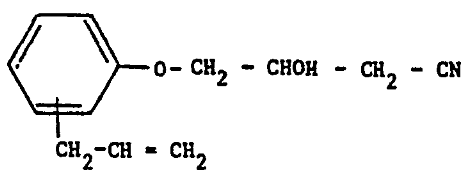

(II)

avec l'hydroxylamine, préférablement dans un milieu réactionnel eau-alkanol inférieur en $C_1$-$C_4$, pendant au moins une heure, à la température de reflux dudit milieu réactionnel, avec un excès d'hydroxylamine par rapport aux conditions stœchiométriques.

En pratique on utilisera plus d'une mole (avantageusement on prendra environ 2 moles) de $NH_2OH$ pour une mole de nitrile II, d'une part, et un milieu eau-alcool (0,5 : 1) à (1 : 0,5) v/v, d'autre part, l'alcool préféré étant le n-butanol.

Les isomères optiques d'un composé de formule I peuvent être isolés selon une méthode connue en soi pour la résolution des énantiomères à partir notamment des racémiques.

Selon l'invention on préconise enfin une composition thérapeutique utile notamment dans le

traitement des troubles du rythme cardiaque et qui est caractérisée en ce qu'elle renferme, en association avec un excipient physiologiquement acceptable, au moins un composé de formule I ou l'un de ses sels non toxiques. Bien entendu, une telle composition renfermera une quantité pharmaceutiquement efficace d'ingrédient actif.

L'exemple de préparation qui suit n'est pas limitatif mais illustre le meilleur mode de mise en œuvre pour la synthèse.

Préparation :

Obtention du chlorhydrate de 4-(2-allylphénoxy)-3-hydroxy-butyramidoxime :

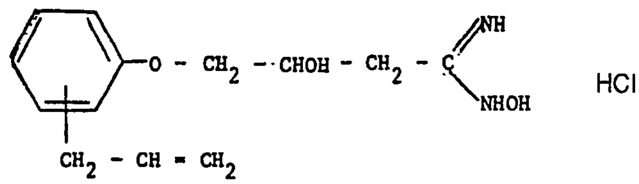

(N° de code : CRL 40708)

a) 1-(o-allylphénoxy)-3-chloro-2-propanol

Dans un ballon on place 92,5 g (I mole) d'épichlorhydrine, 33,5 g (0,25 mole) d'o-allylphénol et l'on ajoute 0,3 ml de pyridine. On porte l'ensemble à 80 °C en 1 h environ puis on élève la température jusqu'à 90 °C que l'on maintient pendant environ 6 h jusqu'à disparition complète du phénol. Le mélange est alors refroidi et l'excès d'épichlorhydrine évaporé. L'huile qui reste est reprise avec 100 ml de chloroforme et additionnée lentement de 50 ml d'HCl ($d_4^{15}$ = 1,19). La phase organique est alors lavée 2 à 3 fois à l'eau puis séchée sur $Na_2SO_4$. Après évaporation du chloroforme, on recueille 56,6 g (rendement : 100 % environ) de 1-(o-allylphénoxy)-3-chloro-2-propanol sous la forme d'une huile de couleur marron foncé.

b) 4-(o-allylphénoxy)-3-hydroxy-butyronitrile

On dissout l'huile précédemment obtenue (≃ 0,25 mole) dans 150 ml de méthanol et additionne au reflux, goutte à goutte, une solution de 22,75 g (0,35 mole) de cyanure de potassium dans 35 ml d'eau. Une fois l'addition terminée, on poursuit le reflux 1 h environ, puis on refroidit, filtre, évapore le méthanol, reprend l'huile obtenue au toluène, lave plusieurs fois à l'eau jusqu'à pH neutre, sèche et évapore sous vide. On recueille 48,9 g (rendement 90 %) de 4-(o-allylphénoxy)-3-hydroxy-butyronitrile.

c) 4-(o-allylphénoxy)-3-hydroxy-butyramidoxime

On prépare une solution aqueuse d'hydroxylamine à partir de 39,06 g (0,562 mole) de chlorhydrate d'hydroxylamine dans 150 ml d'eau et de 56,2 g (0,562 mole) de bicarbonate de potassium. On ajoute alors 48,9 g (0,225 mole) de 4-(o-allylphénoxy)-3-hydroxy-butyronitrile préalablement dissous dans 250 ml de n-butanol. On porte le mélange résultant à 80 °C en 2 h environ, puis on porte au reflux (94 °C) que l'on maintient pendant 5 h. On refroidit, évapore le mélange $H_2O$-$CH_3(CH_2)_2CH_2OH$, reprend le résidu d'évaporation à l'éther, lave plusieurs fois à l'eau, sèche et évapore l'éther sous pression réduite. On obtient 48,5 g (rendement 86 %) de 4-(o-allyl-phénoxy)-3-hydroxy-butyramidoxime sous la forme d'une huile de couleur marron.

d) CRL 40708

On précipite le chlorhydrate par addition d'éthanol chlorhydrique à une solution de la base libre (obtenue comme indiqué ci-dessus) dans de l'acétate d'éthyle. Pour faciliter la précipitation on ajoutera avantageusement un peu d'éther. Après recristallisation dans l'acétate d'éthyle, on obtient le CRL 40708 (avec un rendement de 70 %). F inst. = 94 °C.

En procédant comme indiqué ci-dessus on obtient à partir des m- et p-allylphénols, les isomères m et p du CRL 40708.

On a résumé les essais qui ont été entrepris avec le CRL 40708 selon le protocole suivant : des cobayes (6 animaux par dose) anesthésiés à l'uréthane reçoivent par injection I.V. le CRL 40708 (les animaux témoins reçoivent une injection I.V. de sérum physiologique), cinq minutes après, ils reçoivent par perfusion I.V. soit le nitrate d'aconitine soit la K-strophantine.

On observe que, aux doses de 5 mg/kg, 10 mg/kg et 20 mg/kg I.V., le CRL 40708 retarde l'apparition de tous les accidents de l'électrocardiogramme provoqués par le nitrate d'aconitine et la K-strophantine, et est bradycardisant notamment à la dose de 20 mg/kg I.V.

On retrouve après administration du CRL 40708 par voie orale et par voie intraduodénale les propriétés antiarythmisantes observées après administration intraveineuse. On constate en effet que (i) le CRL 40708 aux doses de 25 mg/kg P.O. et 50 mg/kg I.D. retarde de façon significative l'apparition de tous les accidents de l'électrocardiogramme provoqués chez le cobaye anesthésié par le nitrate d'aconitine et la K-strophantine, et (ii) à la dose de 25 mg/kg I.D. il retarde de façon significative l'apparition de la fibrillation ventriculaire.

On observe également que par voie I.D. le CRL 40708 se distingue du LL 1530 précité qui est un antiarythmisant de référence. Par voie I.D., la dose minimale antiarythmisante du CRL 40708 est voisine de 25 mg/kg alors que le LL 1530 est encore inactif à 25 mg/kg.

En clinique, on a obtenu de bons résultats dans le traitement des troubles du rythme cardiaque chez l'homme par administration de CRL 40708 en tant qu'agent antiarythmisant sous forme de comprimés ou gélules (renfermant chacun 75 à 150 mg de CRL 40708) à raison de 3 à 6 comprimés ou gélules par jour, pendant au moins 1 semaine.

**Revendications** (pour les Etats contractants : BE, CH, DE, FR, GB, IT, LI, LU, NL, SE)

1. Nouveau dérivé de 4-phénoxy-3-hydroxy-butyramidoxime, caractérisé en ce qu'il est choisi parmi l'ensemble constitué par

(i) les 4-(allylphénoxy)-3-hydroxy-butyramidoximes de formule

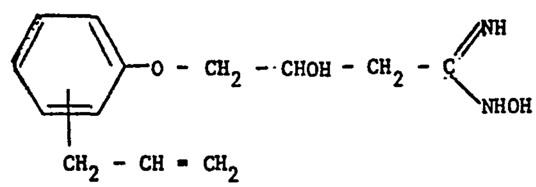

(I)

et

(ii) leurs sels d'addition.

2. 4-(2-allylphénoxy)-3-hydroxy-butyramidoxime et ses sels d'addition.

3. Composition thérapeutique, utile notamment dans le traitement des troubles du rythme cardiaque, caractérisée en ce qu'elle renferme, en association avec un excipient physiologiquement acceptable, au moins un composé de formule I selon la revendication 1 ou l'un de ses sels non toxiques.

4. Procédé de préparation d'un composé de formule I selon la revendication 1, caractérisé en ce que l'on fait réagir l'hydroxylamine avec un 4-(allylphénoxy)-3-hydroxy-butyronitrile de formule

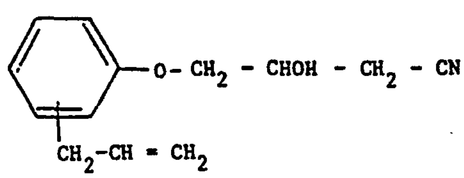

(II)

5. Procédé selon la revendication 4, caractérisé en ce que l'on fait réagir 1 mole de nitrile II avec plus d'une mole d'hydroxylamine, dans un milieu réactionnel eau-alkanol en $C_1$-$C_4$, pendant au moins 1 h à la température de reflux.

**Revendications** (pour l'Etat contractant AT)

1. Procédé de préparation d'un nouveau composé appartenant à la famille des 4-(allylphénoxy)-3-hydroxy-butyramidoximes de formule générale

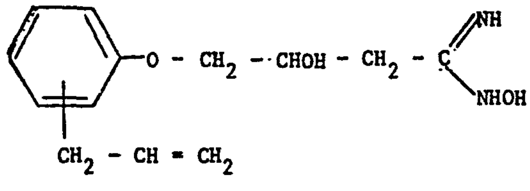

(I)

et de leurs sels d'addition, caractérisé en ce que l'on fait réagir une mole d'un 4-(allylphénoxy)-3-hydroxy-butyronitrile de formule

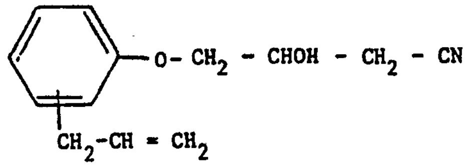

(II)

avec plus d'une mole d'hydroxylamine, dans un milieu réactionnel eau-alkanol en $C_1$-$C_4$, pendant au moins 1 h à la température de reflux.

2. Procédé selon la revendication 1 pour la préparation de 4-(2-allylphénoxy)-3-hydroxy-butyramidoxime et ses sels d'addition, caractérisé en ce que l'on fait réagir l'hydroxylamine avec le 4-(2-allylphénoxy)-3-hydroxy-butyronitrile.

**Claims** (for the Contracting States : BE, CH, DE, FR, GB, IT, LI, LU, NL, SE)

1. A new 4-phenoxy-3-hydroxybutyramidoxime derivative selected from the group consisting of
(i) 4-(allylphenoxy)-3-hydroxy-butyramidoximes of the general formula

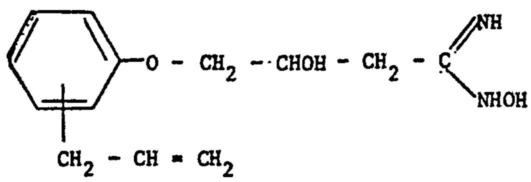

(I)

and,
(ii) addition salts thereof.

2. 4-(2-allylphenoxy)-3-hydroxybutyramidoxime and its addition salts.

3. A therapeutical composition, particularly useful in the treatment of disorders of heart beat rate, characterized in that it comprises, in association with a physiologically acceptable excipient, at least a compound of formula I according to claim 1 or one of its non toxic addition salts.

4. A method of preparation of a compound according to claim 1, characterized in that hydroxylamine is reacted with a 4-(allylphenoxy)-3-hydroxy-butyronitrile of the formula

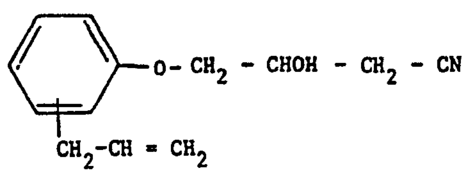

(II)

5. A method of preparation according to claim 4, characterized in that 1 mol of nitrile II is reacted with more than 1 mol of hydroxylamine in a $C_1$-$C_4$-alkanol-water medium, for at least 1 hour under refluxing.

**Claims** (for the Contracting State AT)

1. Process for the preparation of a new compound belonging to the family of 4-(allylphenoxy)-3-hydroxy-butyramidoximes of the general formula

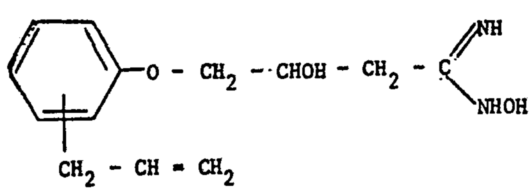

(I)

and their addition salts thereof, characterized in that a mol of 4-(allylphenoxy)-3-hydroxy-butyronitrile of the formula :

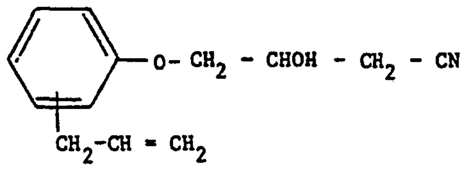

(II)

is reacted with more than one mol of hydroxylamine in a $C_1$-$C_4$-alkanol-water reaction medium, for at least one hour under refluxing temperature.

2. Process according to claim 1, for the preparation of 4-(2-allylphenoxy)-3-hydroxybutyramidoxime and its addition salts, characterized in that hydroxylamine is reacted with 4-(2-allylphenoxy)-3-hydroxy-butyronitrile.

**Ansprüche** (für die Vertragsstaaten : BE, CH, DE, FR, GB, IT, LI, LU, NL, SE)

1. Neues 4-Phenoxy-3-hydroxy-butyramidoxim-Derivat, dadurch gekennzeichnet, daß es ausgewählt ist aus der Gruppe bestehend aus

(i) den 4-(Allylphenoxy)-3-hydroxy-butyramidoximen der Formel

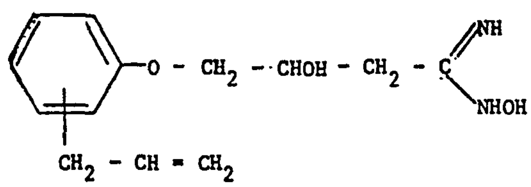

(I)

(ii) ihren Additionssalzen.

2. 4-(2-Allylphenoxy)-3-hydroxy-butyramidoxim und seine Additionssalze.

3. Therapeutische Zusammensetzung, wertvoll insbesondere bei der Behandlung von Herzrhythmusstörungen, dadurch gekennzeichnet, daß sie in Verbindung mit einem physiologisch akzeptablen Exzipienten zumindest eine Verbindung der Formel I nach Anspruch 1 oder eines ihrer nicht toxischen Salze enthält.

4. Verfahren zur Herstellung einer Verbindung der Formel I nach Anspruch 1, dadurch gekennzeichnet, daß Hydroxylamin mit einem 4-(Allylphenoxy)-3-hydroxy-butyronitril der Formel

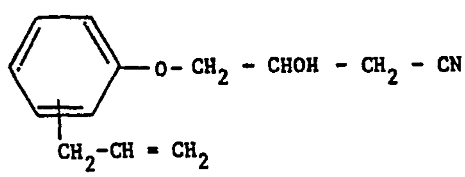

(II)

zur Reaktion gebracht wird.

5. Verfahren nach Anspruch 4, dadurch gekennzeichnet, daß 1 Mol Nitril II mit mehr als einem Mol Hydroxylamin in einem Reaktionsmedium aus Wasser-$C_1$-$C_4$-Alkanol zumindest 1 h bei Rückflußtemperatur zur Umsetzung gebracht wird.

**Ansprüche** (für den Vertragsstaat AT)

1. Verfahren zur Herstellung einer neuen Verbindung, die zur Familie der 4-(Allylphenoxy)-3-hydroxy-butyramidoxime der allgemeinen Formel

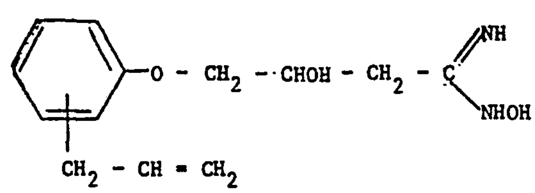

(I)

gehört, und ihrer Additionssalze, dadurch gekennzeichnet, daß ein Mol eines 4-(Allylphenoxy)-3-hydroxy-butyronitrils der Formel

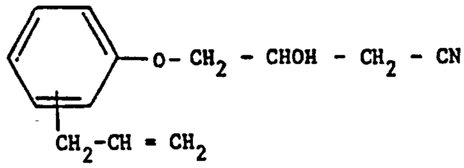

(II)

mit mehr als einem Mol Hydroxylamin in einem Reaktions-medium aus Wasser-$C_1$-$C_4$-Alkanol zumindest 1 h bei Rückflußtemperatur zur Umsetzung gebracht wird.

2. Verfahren nach Anspruch 1 zur Herstellung von 4-(2-Allylphenoxy)-3-hydroxy-butyramidoxim und seiner Additionssalze, dadurch gekennzeichnet, daß Hydroxylamin mit 4-(2-Allylphenoxy)-3-hydroxy-butyronitril zur Umsetzung gebracht wird.